# EUROPEAN PATENT APPLICATION

(11) **EP 3 903 764 A1**
(43) Date of publication of application: **03.11.2021**
(21) Application number: 18945307.9
(22) Date of filing: 28.12.2018
(51) Int. Cl.: A61K 8/19, A61K 8/21, A61K 8/24, A61Q 11/00

(54) **MULTIPURPOSE FLUORINATED BIPHOSPHATE TOOTHPASTE COMPOSITION**

(71) Applicant: Teixeira, Marcelo Rodrigues, 86055-736 Londrina - PR (BR); Rabbit Indústria e Comércio de Produtos de Higiene Pessoal Ltda, 86046-140 Londrina - PR (BR)
(72) Inventor: FABIANO VIEIRA, Vilhena, 12243-001 São José dos Campos - SP (BR)
(74) Representative: Patentree
(86) International application number: PCT/BR2018/050485
(87) International publication number: WO 2020/132720

(57) **Abstract**

The present invention describes a dentifrice composition for daily use, with the capacity of promoting antidemineralizing, regenerative and desensitizing actions, relieving pains and promoting the complete maintenance of the oral health. For this purpose, there is comprised a small quantity of water (less than 5%), a fluoridated agent (sodium fluoride), and two phosphate compounds (tetrasodium pyrophosphate (Na₄P₂O₇) together with phosphoric acid (H₃PO₄)), which grant to it antidemineralizing/regenerative action. When in contact with the minerals of the tooth, the composition prevents the loss of calcium and phosphate during the process of demineralization, while at the same time promoting the repair of areas that are already demineralized in initial stage, at the enamel level. The composition further has dentinal desensitization action, due to the capacity of precipitation of CaF₂, which occludes the dentinal tubules. In this manner, the present invention contributes to the art by providing in one sole product the maintenance and complete treatment of the oral health.

## Description

### BRIEF DESCRIPTION

The present patent application refers to a unique **MULTIPURPOSE FLUORINATED BIPHOSPHATE TOOTHPASTE COMPOSITION** which describes a dentifrice formulation, comprising a synergistic association of a small quantity of water, a fluoride agent, and two phosphate compounds, which grant an antidemineralizing/regenerating action to the composition which, in contact with the minerals of the teeth, avoids the loss of calcium and phosphate, that occurs during the demineralizing process, at the same time that it promotes the repair of areas already in an initial stage of demineralization, that is, at the of tooth enamel level. The composition further comprises tooth desensitizing action, due to the capacity thereof of precipitation of CaF₂, which occludes the dentinal tubules.

### FIELD OF APPLICATION

The present invention belongs to the sector of human needs, to the field of medical science, dental and hygiene; more specifically, to dental preparations, by describing a dentifrice formulation, which comprises a synergistic association of the components thereof, granting antidemineralizing, regenerative and desensitizing action to the composition.

### ARGUMENTATION

Maintaining oral health updated is synonym to quality of life and brings results to the physical, mental and social well-being of the individual. In the oral cavity, the disease is expressed in several manners, whereby the most known one is: dental carie. Throughout history, the occurrence of dental carie increased considerably since the Industrial Revolution - resulting from processed foods using sugar. Currently, behavior factors in society, for example, stress, result in the appearance of new oral health problems, among them the dental erosion and dentinal sensitization. An important ally in the prevention of oral problems, is the self-care with oral hygiene products, therefore it is exactly within this context that the present invention is inserted.

Formulations for oral cleansing are dated from centuries before Christ. Nowadays, the worry with oral hygiene comes linked with the rhythm of modern society, therefore, much more is demanded than just cleaning, aiming at the maintenance of oral health. In this context the current formulations aggregate antimicrobial effects, anticaries, antiplaque, anti-pain, among others.

As regards the state of the art, dentifrice formulations, in general lines, have as their components: humectants, thickeners, vehicle, sweeteners, preservatives, abrasives, surfactants, flavorings and active principles. Whereby the main differences between the toothpastes, are exactly the ones responsible for combatting the main oral problems.

Caries and acid erosion are the main problems faced in the context of destructive processes of the dental tissue, also called demineralization. To combat these problems, fluorine has become the main active agent incorporated.

According to the most recent revision on the matter (Leal & Takeshita et al 2018 - Pediatric Restorative Dentistry*),* the mechanisms by means of which fluoride affects the demineralization and remineralization of the enamel and the dentine are already well established, being known for depending on calcium and phosphate infraoral ions available in the presence of fluorine. Phosphate based agents have been investigated on their own or associated with fluorine, which results in greater protection and prevention against the demineralization. In general lines, the phosphate compounds help in the formation of the tooth mineral, the hydroxyapatite. Some of these compounds are used as calcium and phosphate sources, to increase the saturation of the oral environment as regards the hydroxyapatite, which includes: nanohydroxyapatite, bioactive glass containing sodium and calcium phosphosilicate (NovaMin®), double chamber system wherein the calcium salts are separated by the plastic compartment from phosphate salts and sodium fluorine (Enamelon®), dicalcium phosphate dihidrate (DCPD), caseinophosphopeptides (CPP) linked to the amorphous calcium phosphate (ACP) (Recaldent®), functionalized tricalcium phosphate and calcium glycerophosphate.

Still in accordance with Leal & Takeshita et al., 2018 (Pediatric Restorative Dentistry)*,* apart from these compounds, the pH of the dentifrice formulation also appeared to be equally important for the remineralization.

The control of the pH, provided by the phosphoric acid, showed itself to be the most adequate, since the presence of phosphate together with the acidulated pH is essential for the demineralization and remineralization process (Leal & Takeshita *et al.,* 2018). In this sense, much has been said about the acidulated toothpastes, for nearly fifty years, as observed in table 1, as follows:

**Table 1: Studies related to toothpastes in the last 50 years. Extracted and translated from the book pediatric restorative dentistry - leal & takeshita et al. 2018.**

| Authors (publication year) | Study protocol | Main variables studied | Main results |
|---|---|---|---|
| Gerdin (1974) | *in vivo* | Increase of caries (dmfs) | 250 ppm F (pH 5,5) similar to 1000 ppm F as regards increase of carie |
| Petersson *et al.* (1989) | *in vitro* | Absorption of fluorine in the enamel | 250 ppm F (pH 5,5) similar to 1000 or 1500 ppm (neutral pH) |
| Negri & Cury (2002) | *in vitro* | Absorption of fluorine in the enamel | 550 ppm F (pH 5,5) similar to positive control (1100 ppm F, neutral pH) as regards the fluorine with strong and weak ligands |
| Brighenti *et al.* (2006) | *in vitro* | Demineralization of the enamel | 550 ppm F (pH 5,5) similar to 1100 ppm F (neutral pH) |
| Alves *et al.* (2007) | *in vitro* | Demineralization of the enamel | 412 or 550 ppm F (pH 4,5) similar to 1100 ppm F (neutral pH) |
| Nobre dos Santos *et al.* (2007) | *in situ* | Remineralization of enamel/absorption of fluorine in the enamel | 550 ppm F (pH 5,5) similar to 1100 ppm F as regards the remineralization of the enamel and fluorine strongly connected. |
| Olympio *et al.* (2007) | *in vivo* | Salivary fluorine concentration | Toothpaste 550 (pH 5,5) containing 550 ppm of high concentrations of salivary fluorine at levels similar to the positive control. |
| Buzalaf *et al.* (2009) | *in vitro* | Enamel wear (abrasiveness) | 550 ppm F (pH 4,5) similar to 1100 ppm F (neutral pH) as regards the fluorine concentrations in the dental biofilm. The reduction of the pH of the dentifrice did not affect the concentration of nail fluoride (that is, systemic effect). |
| Vilhena *et al.* (2010) | *in vivo* | Progression of carie (dmfs) | 550 ppm F (pH 4,5) similar to 1100 ppm F (neutral pH) |
| Brighenti *et al.* (2013) | *in vitro* | Remineralization of the enamel | 550 ppm F (pH 4,5) similar (surface hardness) or greater (cross-sectional hardness) to 1100 ppm F (neutral pH) |
| Moron *et al.* (2013) | *in vitro* | Enamel wear (erosion) | Less protective effect from 550 ppm F (pH 4,5) compared to 1100 ppm F (neutral pH) |
| Cardoso *et al.* (2014) | *in vivo* | Progression of caries and regression/fluorine concentration in toenails | Carie progression significantly lower and liquid increase to 550 ppm (pH 4,5) compared with 1100 ppm F neutral (Nyav criteria). 550 ppm F (pH 4,5) showed a significantly improved performance than the neutral of 1100 ppm F (QLF analysis). Lower concentration of fluorine in toenail associated to the toothpaste with low fluorine content (that is, systemic effect). |
| Cardoso *et al.* (2015) | *in vitro in vivo* | Demineralization of enamel /Absorption of fluoride in the biofilm | Lower effect of 550 ppm F (pH 4,5) in the demineralization of enamel in comparison with 1100 ppm neutral F; 550 ppm F (pH 4,5) promoted a significantly higher absorption of biofilm fluorine in comparison with the 1100 ppm neutral F. |
| Kondo *et al.* (2016) | *in vivo* | Levels of fluorine in saliva and biofilm (solid and fluid phases) | Higher concentrations of fluorine in biofilm 1 hour after the brushing with acid dentifrices in comparison with the neutral counterparts, although the differences were not significant; the pH of the toothpaste did not affect the concentrations of saliva fluoride. |
| Ortiz *et al.* (2016) | *in vitro* | Demineralization of enamel and absorption of fluorine in the enamel. | 550 ppm F (pH 4,5) effect similar or greater than 1100 ppm F (neutral) in relation to the fluorine with strong and weak ligands; lower effect against demineralization |
| Veloso *et al.* (2017) | *in vivo* | Absorption of fluorine in the biofilm | 750 ppm F (pH 4,5) similar to neutral 1100 ppm F 60 minutes after brushing |
| Campos *et al.* (2017) | *in vivo* | Concentration of fluorine in the toenails | 750 ppm F (pH 4,5) led to lower fluorine levels in toenails than the 1100 ppm neutral F |

The sensitivity in teeth affects several people causing pains and discomfort, however, the treatment thereof is possible and relatively simple, by means of the daily use of specialized toothpastes, which are capable of reducing the pains and bringing relief. Thus, several formulas are known for the treatment of dentinal hypersensitivity. Developed as from scientific researches, three substances are noted among the active principles that are most used in these products: potassium nitrate, which prevents the nerves from transmitting pain messages to the brain; strontium acetate, which temporarily hinders the arrival of fluids in the passages of the tooth that give access to the pulp; and NovaMin®, which scientific name is calcium and sodium phosphosilicate, which promotes the continuous remineralization (April - Super Interessante, 2017).

Evidently it is possible to obtain similar or greater effects when compared with the conventional fluorine products. However, an ideal formulation must combine the capacity of improving the remineralization and reducing the mineral loss, the erosive wear and the dentinal sensitivity (Leal & Takeshita et al., 2018 - Pediatric Restorative Dentistry*).*

Current dentifrice formulations need to provide complete protection for the mouth, that is, they must contain the function of prevention, protection and repair against carie/dentinal erosion, as well as the remaining oral problems, while at the same time relieving and preventing pain, that is, the sensitivity in the teeth.

According to Dheeraj and collaborators (Nonfluoride remineralization: An evidence-based review of contemporary Technologies, 2014), the ideal requirements for the activity of dental remineralization are: spread out on the sub-surface, providing calcium and phosphate on the sub-surface; not providing excess calcium; not forming dental calculus; acting in environment with acid pH; working on xerostomic patients, and; increasing the remineralizing properties in the saliva.

In view of the above, the present invention brings a formulation which completely meets the maintenance of oral health, solving the problem present in the current state of the art, as regards the proposal of a dentifrice composition with capacity of promoting maintenance of oral health, in a complete manner, in one sole product.

### BACKGROUND OF THE INVENTION

In the current state of the art there are present prior art that describe dentifrice compositions which aim at improving sensitivity and dentinal remineralization. However, there are not found compositions which, in one sole product, can provide total maintenance of the oral health, by means of a synergistic association of a small quantity of water, a fluoridated agent and two phosphate compounds, which provide antidemineralization/regenerating action, while at the same time providing the repair of the area that is already demineralized in initial stage, being further, a desensitizing composition.

Prior art PI0705195-6, entitled "Acidulated dentifrice liquid formulation having low concentration of fluorine and use thereof", describes a "liquid" (fluid gel) dentifrice formulation, consisting of fluorine having a content lower than or equal to 750 ppm, from 4 to 50% abrasives, from 20 to 65% humectants, from 0,5 to 7% agglutinants, 1 to 6% surfactants, from 0,01 to 0,5% preservatives, from 0 to 750 ppm of fluoridated agents, from 0,25 to 2% antiplaque agents and acidulant agents in a proportion that guarantees a pH of 4,3 to 6,0. The proposal of a low concentration of fluorine and reduced pH, aims at increasing the efficacy of the product in the control of the dental carie and lessen the risk of occurrence of dental fluorosis.

The pH adjustment of the prior art was facilitated by the higher concentration of water which, in the presence of the acid, promotes the hydrolysis, forming hydronium, having a lower pH (-1,7 pKa) than the acid itself (2,7 pKa). The hydronium contributes for the ideal pH (4,5) to be reached. The hydrolysis occurs when a salt having a weak base or a weak acid (or both) is dissolved in water. The presence of little water in the present invention favored the stability of the phosphate compounds until the use thereof in the oral cavity where, when in contact with the saliva, which is rich in calcium, it promoted the precipitation of fluorine, which linked with the saliva calcium, originating calcium fluoride. Thus, in the present invention there occurred an increase in the fluorine concentration, great water reduction and great increase of acid for the correction of the pH to around 4,5.

Although the gels described in the prior art present the same initial pH, 4,5, after 1 minute brushing the pH of the gel did not sustain itself so acid, that is, after this time, the pH rose to around 6,5. Which shows one more advantage of the present invention as regards the state of the art, since the ideal pH range, in the mouth, for the mineralization, occurs as from 5,5 and that, although this prior art presents pH that is numerically close, this difference is quite significant in the final result of the remineralizing capacity, due to the higher permeability, that is, larger penetration of the minerals in the enamel. Thus, the reaction that sustains the more acid pH in the mount, and which occurs in the invention, results due to the contact with the water (saliva), which promotes the hydrolysis of the acid of the composition, forming hydronium, having a lower pH (-1,7 pKa) than the acid itself (2,7 pKa).

Prior art BR102013006807-1, entitled "Ultrafunctional dentifrice and use of the association of a fluoridated agent and protease inhibitor", describes a dentifrice formulation containing fluoride and protease inhibitors, having reduced pH, between 4,3 and 6; to increase the efficacy of the product in the control of dental carie, dental acid erosion and periodontal disease. Although the prior art describes the use of phosphoric or orthophosphoric acid, as in the present invention, as an acidulant agent, the pH of the present invention is more acid and specifically adjusted for the composition to be able to promote remineralization and dentinal desensitization. For this purpose, the present invention proposes the increase in the fluorine concentration and a great reduction in water, which promotes the increase of the acid for the correction of the pH, which is maintained around 4,5.

Prior art US4997640, entitled *"Oral composition",* describes the use of ascorbic acid as acidulant agent in its composition, to obtain an acid pH, between 4-5. Distinctly from the present invention, the use of the acidulant agent is directly and only related to the adjustment of the pH of the solution, while in the present invention, apart from the role of pH adjustment, the orthophosphoric acid has the function, together with the tetrasodium pyrophosphateof making phosphate available in the composition.

The increase in the phosphorus concentration with the increase in the fluorine concentration in the dentifrices is directly related to the fact that the addition of NaF increases the pH of the product to around 8,0. Thus, a larger quantity of phosphoric acid is necessary to adjust the pH at 4,5 in dentifrices having more than 1000 ppmF, as reported by Brighenti *et al.* 2006 and 2013.

The present invention has a high fluorine concentration (1450 ppm F), together with a high concentration of phosphate, it is a bisphosphate composition, which has orthophosphoric acid and tetrasodium pyrophosphate (in a proportion of around 2%, added together) in acidulated pH and with very little water. In this context, the present invention brings a higher concentration of stable phosphates in acid medium, due to the small presence of water, promotes a higher instantaneous precipitation of calcium fluoride, when in contact with the tooth and the saliva. This precipitation is responsible for the occlusion of the dentinal tubules (remineralization of the carious process and dentinal desensitization). In prior technologies, NovaMin® for example, despite the action of the product being the same, that is, promoting occlusion of the dentinal tubules, the present invention brings a different and innovative process for this to occur, which distinguishes the present invention from the state of the art, bring contribution to the art.

The combination of the components that promotes a joint action of regeneration and dentinal desensitization (anti-pain), described in the present invention, is explained by the immediate precipitation of the calcium fluoride, mineral precursors of the tooth (hydroxyapatite). For this to occur in a satisfactory manner, it is necessary: that the quantity of water present in the formulation be less than 5%, to favor the larger quantity of orthophosphoric acid in the pH adjustment; the acidulant agent, the orthophosphoric acid, have a double function to guarantee the final pH of the product between 4,3 and 5,3 and, at the same time, provide phosphate in the formulation (together with the tetrasodium pyrophosphate) for the precipitation of calcium fluoride; have high fluorine concentration, so that there is a higher formation of fluorapatite; have the tetrasodium pyrophosphate, a phosphate compound which acts as anti-tartar agent, remineralizer and desensitizer. Thus, with the increase in the concentration of phosphate compounds, there is promoted a joint regenerative and anti-pain, therefore a regeneration and a dentinal desensitization, which is called a *Refix* technology.

Although the state of the art contains prior art that describe dentifrice compositions capable of bringing pain relief and promoting the remineralization of the teeth, none of them brings a formulation which meets in a complete manner the maintenance of the oral health, by means of the use of one sole product. In the following table, a brief comparison is made of the invention described in the present application with dentifrices that are present in the state of the art, summarizing their main differences:

**Table 2: Comparison between the invention and the dentifrices of the state of the art.**

| | Invention | Dentifrices present in the state of the art | |
|---|---|---|---|
| | | Acid pH dentifrices | Neutral/base pH dentifrices |
| In tube (Formulation) | - Water | + Water | + Water |
| | ++ Acid | Acid | Without acid |
| | + Phosphate | Phosphate | ± Phosphate |
| | pH 4,5 | pH 4,5 | pH 7,0 or higher |
| | | | |
| In mouth | + Phosphate | Phosphate | ± Phosphate |
| | pH 5,5 | pH 6,5 | pH 7 or higher |
| | ++ FCa₂ Phosphate | + FCa₂ Phosphate | ± FCa₂ Phosphate |
| | | | |
| In tooth | + Enamel permeability | Enamel permeability | Enamel permeability |
| | + mineral precipitation | mineral precipitation | ± mineral precipitation |
| | | | |
| Results | + Remineralization | Remineralization | ± Remineralization |
| | + Desensitization | - | ± Desensitization |

### OBJECTIVE OF THE INVENTION

The present invention has the objective of providing a dentifrice composition for daily use, with the capacity of promoting antidemineralizing, regenerative and dentinal desensitizing action, relieving pains and promoting maintenance of the complete oral health.

### ON THE INVENTION

The present invention describes a dentifrice formulation which comprises the synergistic association of a small quantity of water (less than 5%), a fluoridated agent (sodium fluoride) and two phosphate compounds (tetrasodium pyrophosphate (Na₄P₂O₇) together with phosphoric acid (H₃PO₄)), which grant it antidemineralizing/regenerative action. When in contact with the minerals of the tooth, the composition prevents the loss of calcium and phosphate during the demineralization process, while at the same time it promotes the repair of the area that is already demineralized in initial stage, at the enamel level. The composition further comprises a dentinal desensitization action due to the capacity of precipitating the CaF₂, which occludes the dentinal tubules.

### ADVANTAGES OF THE INVENTION

The present invention presents as the main advantages:
✔ Providing a dentifrice composition for daily use having a reduced water percentage, less than 5%, which favors the larger quantity of orthophosphoric acid used in the pH adjustment;
✔ Providing a dentifrice composition for daily use, wherein the orthophosphoric acid has two functions, of guaranteeing an acid pH, between 4,3 and 5,3, while at the same time it provides phosphate to the composition, together with the tetrasodium pyrophosphate, for the precipitation of calcium fluoride, which promotes the occlusion of the dentinal tubules.
✔ Providing a dentifrice composition for daily use having higher fluorine concentration, which enables a larger formation of calcium fluoride and fluorapatite;
✔ Providing a dentifrice composition for daily use having an anti-tartar agent, the tetrasodium pyrophosphate (phosphate compound), which acts as collaborator in the remineralization and dentinal desensitization process;
✔ Providing a dentifrice composition for daily use having a bactericide agent, triclosan, which contributes to the dentinal demineralization;
✔ Providing a dentifrice composition for daily use with incorporation of sweetener, xylitol, which contributes to the dentinal remineralization process.

### DESCRIPTION OF THE FIGURES

The invention will be described in a preferred embodiment, thus, for a better understanding, reference will be made to the figure:

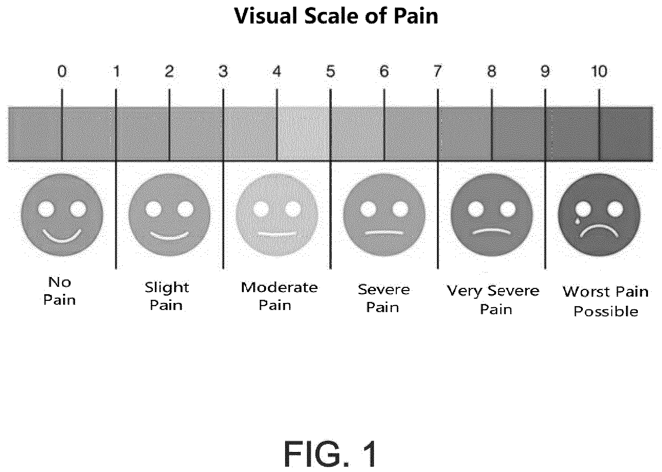

**Figure 1****:** Visual analogue scale.

### DETAILED DESCRIPTION OF THE INVENTION

Prior to obtaining the dentifrice composition itself, a sample of 100 ml of deionized water is reserved for quality control and pH analysis, next, all the components of the composition are segregated and weighed, according to table 3, as follows:

**Table 3: Components used to obtain the dentifrice composition.**

| Components of the formula | Quantity % |
|---|---|
| Humectant (glycerin, Sorbitol, polyethylene glycol - PEG 600) | 50 to 65 |
| Thickener (Carboxymethylcellulose, thickener silica) | 8 to 12 |
| Vehicle (deionized water) | 2 to 4,9 |
| Active -Anticaries agent (Sodium fluoride) | 0,25 to 0,5 |
| Sweetener (Sodium saccharin Xylitol) | 0,5 to 1,5 |
| Preservative (Sodium benzoate) | 0,1 to 0,5 |
| Active - anti-tartar agent (Tetrasodium pyrophosphate) | 0,3 to 1 |
| Abrasive (Abrasive silica) | 5 to 15 |
| Surfactant (Sodium lauryl sulfate, vacuum) | 5 to 10 |
| Antiseptic (Triclosan) | 0,1 to 0,3 |
| Flavoring | 1 to 3 |
| Pigment (Mica) | 0,1 to 0,2 |
| Active - pH corrector (Orthophosphoric acid) | 0,8 to 2 |

Then, there begins the preparation of a pre-mix, wherein the glycerin + CMC phase is obtained, for which there are used:
✔ Carboxymethylcellulose, in a proportion from 0,7 to 1,0% m/m of the composition;
✔ Glycerin, in a proportion from 45 to 55% m/m of the composition.

The carboxymethylcellulose is added to the glycerin, under slow agitation, between 45.000 to 200.00 rpm at 25°C, to avoid forming lumps, whereby the agitation continues until the complete dissolution of the carboxymethylcellulose, which takes around 15 minutes, and forming of the glycerin + CMC phase.

Subsequently, in a reactor the following components are added to be homogenized with turbine, scraper and propeller, for a period of 10 minutes, at the speed and temperature indicated by the manufacturer of the equipment:
✔ Deionized water, in a proportion from 2 to 4,9% m/m of the composition;
✔ Sodium fluoride, in a proportion from 0,25 to 0,5% of the composition;
✔ Sodium saccharin; in a proportion from 0,2 to 0,5% m/m of the composition;
✔ Sodium benzoate, in a proportion from 0,1 to 0,5% m/m of the composition;
✔ Xylitol, in a proportion from 0,3 to 1% m/m of the composition;
✔ Tetrasodium pyrophosphate, in a proportion from 0,3 to 1,0% m/m of the composition.

Next, the following are added to the mixture, to be homogenized with turbine, scraper and propeller, for a period of 10 minutes, at the speed and temperature indicated by the manufacturer of the equipment:
✔ Sorbitol, in a proportion from 3 to 10% m/m of the composition;
✔ Polyethylene glycol - PEG 600, in a proportion from 0 to 2% m/m of the composition;
✔ Glycerin phase + CMC, previously prepared, in its entirety.

Next, the following are added to the mixture, to be homogenized with turbine, scraper and propeller, for a period of 01h:30 minutes, at the speed and temperature indicated by the manufacturer of the equipment:
✔ Thickener silica, in a proportion from 9 to 11% m/m of the composition;
✔ Abrasive silica, in a proportion from 5 to 15% m/m of the composition.

Next, the following are added to the mixture, to be homogenized with turbine, scraper and propeller, for a period of 10 minutes, at 120°C in the speed indicated by the manufacturer of the equipment:
✔ Sodium lauryl sulfate, vacuum, in a proportion from 5 to 10% m/m of the composition, being used.

Next, add to the mixture, for homogenization with scraper, for a period of 15 minutes, at the speed and temperature indicated by the manufacturer of the equipment:
✔ Triclosan, in a proportion from 0,1 to 0,3% m/m of the composition, as antiseptic agent;
✔ Flavoring, in a proportion from 1 to 3% of the composition;
✔ Mica, in a proportion from 0,1 to 0,2% m/m of the composition.

After obtaining the dentifrice composition, which occurs after the homogenization of all the components, as described, there is carried out the correction of the pH, to a value between 4,5 and 5,0; by means of the addition and homogenization, at the speed and temperature indicated by the manufacturer of the equipment, of orthophosphoric acid, in a proportion from 0,8 to 2,0% m/m of the composition.

Subsequently, the composition is filled in tubes, now denominated daily regenerator gel. For filling, the operator executes weight trials throughout the filling process to verify whether the specified grammature corresponds to what is specified. After filling, the regenerator gel tubes are packaged and labeled, according to guidelines of the regulatory agency.

Additionally, the daily regenerator gel can present in its composition a calcium supplement, since materials containing calcium have the potential to increase the remineralization of the lesions of the dentinal enamel (Exterkate et al. (1993), A Single-section Model for Enamel De- and Remineralization Studies. I. The Effects of Different Ca/P Ratios in Remineralization Solutions*;* and Tanaka et al. (2012), Optimization of calcium concentration of saliva with phosphoryl oligosaccharides of calcium (POs-Ca) for enamel remineralization in vitro*).* In this sense, studies have been carried out to find the best relation of the quantity to be made available, as regards the remaining components of the formulation. Initial results have shown that a calcium concentration, around 0,5 larger than that of phosphate, a relation Ca:P of 1,67 provides an optimum rate of remineralization, which corroborates the data of the literature.

Adding to the remaining components of the formula, which provided a perfect medium, in the mouth and teeth, for forming remineralizing minerals, the calcium fluoride and fluorapatite, a Ca supplement enhances this remineralizing action of the composition described. For this purpose, the calcium supplement can be carried out by means of the addition of calcium salts, in a proportion from 0,5 to 6% m/m of the composition, preferably, using: Calcium Glycerophosphate, Calcium Carbonate and Calcium Hydroxide. Said supplementation may happen by means of the addition directly in the composition (encapsulated or not), or further, as a serum booster, to be added at the moment of use, and found apart in the storage packaging, working as an accelerator/enhancer.

In order to validate the promotion property of the dentinal demineralization, regeneration and desensitization actions, of the daily regenerator gel, there were carried out clinical and laboratory trials, which are presented as follows.

A convenience sample formed by 53 patients of 22 dental surgeons who carry out treatments against dental sensitivity and tooth whitening, received the daily regenerator gel described, to brush their teeth after confirming having felt some sensitivity.

When the patient reported pain due to sensitivity, he filled out a card, indicating the sensitivity level in Visual Analogue Scale - VAS of pain (Figure 1), in a scale from 1 to 10, where 0 means "no discomfort" reaching "severe discomfort" on 10. After indicating in the scale related to the level of pain, the patient was invited to brush his teeth with the daily regenerator gel. Immediately after use, after one minute brushing, the patient made a new indication in the pain scale, following the same procedure. After one week of use of the daily regenerator gel, the patient indicated the scale, following the same procedure.

Thus, for the test, the patients made three markings on the pain VAS: one immediately after the pain, prior to using the daily regenerator gel, one right after the first brushing, for one minute, with the daily regenerator gel, and the third one week after the use of the daily regenerator gel. The average obtained in the first medication prior to using the daily regenerator gel, was 7, which means "severe pain", after the first brushing with the daily regenerator gel, the average was reduced to 3, which means "slight pain"; after one week of use of the daily regenerator gel, the average was reduced even more to 1, which means "no pain".

In the clinical cases, there was evaluated the possibility of having significant differences in the slurry produced in the mouth after 1 minute brushing with the daily regenerator gel, described in the present invention. In comparison with a neutral tooth gel, it was verified that the pH of the slurry reached 5,5 for the invention, and around 7 for the neutral dental gel, after one minute's brushing, which showed that the invention has an ideal pH for the process of remineralization, corroborating with that highlighted by Dheeraj and collaborators (2014) that the good working of the remineralizer tooth paste is found in acid pH.

For the laboratory analysis, there were prepared 8 blocks of bovine enamel which had their enamel surfaces brushed, in a brushing machine, with the toothpastes, using for comparison, namely: Regenerate Enamel Science®, Elmex®, Colgate® Daily Repair and the daily regenerator gel, described in the present invention.

For the experiment, the degree of surface hardness of the blocks was measured prior to the demineralization (baseline), next the blocks were demineralized and a new hardness measurement was carried out. After the second measurement, the blocks were brushed for one week in the brushing machine with each one of the toothpastes. At the conclusion of this process, a third hardness measurement was carried out after brushing the blocks. From the results obtained, it is possible to affirm that the only one that enabled the complete mineral reposition of the teeth, that is, the hardness level equal to that of the baseline, after brushing for one week, was the daily regenerator gel, described in the present invention. In view of the above, it is clearly possible to evidence the technical advance and differential effect developed as regards the state of the art.

From the above, it is observed that the synergistic association of the components of the daily regenerator gel, enables the prevention of the main problems of the mouth; regression of the dental carie; regeneration of the tooth enamel, relief and prevention of pain caused by dental sensitivity; apart from up to 13 hours of protection. Further, as suggested by Dheeraj and collaborators (2014), the xylitol and triclosan, present in the composition, have anti-carie effect and are capable of reverting the carious process *(Nonfluoride remineralization: An evidence-based review of contemporary Technologies).*

In this manner, from the above, it is observed that the MULTIPURPOSE FLUORINATED BIPHOSPHATE TOOTHPASTE COMPOSITION is worthy of the privilege of patent of invention.

## Claims

1. **MULTIPURPOSE FLUORINATED BIPHOSPHATE TOOTHPASTE COMPOSITION** wherein it presents in the constitution thereof:
✔ sodium fluoride, in a proportion from 0,25 to 0,5% m/m of the composition as anti-carie agent;
✔ tetrasodium pyrophosphate, in a proportion from 0,3 to 1,0% m/m of the composition, as anti-tartar agent;
✔ orthophosphoric acid, in a proportion from 0,8 to 2,0% of the composition, as pH regulator;
✔ triclosan, in a proportion from 0,1 to 0,3% m/m of the composition, as antiseptic agent; and
✔ deionized water, in a proportion from 2 to 4,9% m/m of the composition, as vehicle.

2. **MULTIPURPOSE FLUORINATED BIPHOSPHATE TOOTHPASTE COMPOSITION,** according to claim 1, wherein it presents in its constitution, as excipients:
✔ humectant, in a proportion from 50 to 60% m/m of the composition, whereby the agents used are: glycerin, sorbitol, polyethylene glycol 600;
✔ thickener, in a proportion from 8 to 12% m/m of the composition, whereby the agents used are carboxymethylcellulose and thickener silica;
✔ sweetener, in a proportion from 0,5 to 1,5% m/m of the composition, whereby the agents used are: sodium saccharin and xylitol;
✔ preservative in a proportion from 0,1 to 0,5% m/m of the composition, whereby sodium benzoate is used;
✔ abrasive, in a proportion from 5 to 15% m/m of the composition, wherein abrasive silica is used;
✔ surfactant, in a proportion from 5 to 10% m/m of the composition, wherein sodium lauryl sulfate, in vacuum, is used;
✔ flavorings, in a proportion from 1 to 3% m/m of the composition;
✔ pigment in a proportion from 0,1 to 0,2% m/m of the composition, wherein mica is used.

3. **MULTIPURPOSE FLUORINATED BIPHOSPHATE TOOTHPASTE COMPOSITION,** according to any one of claims 1 or 2, wherein it presents a pH between 4,3 and 5,3.

4. **MULTIPURPOSE FLUORINATED BIPHOSPHATE TOOTHPASTE COMPOSITION,** according to any one of claims 1, 2 or 3, wherein it is alternatively supplemented with calcium salts, using a proportion from 0,5 to 6% m/m of the composition, wherein the calcium sources used are preferably, calcium glycerophosphate, calcium carbonate and calcium hydroxide.

5. **MULTIPURPOSE FLUORINATED BIPHOSPHATE TOOTHPASTE COMPOSITION,** according to any one of claims 1, 2, 3 or 4, wherein it is daily applied as antidemineralizing, regenerative and dental desensitizer.
